(19) Europäisches Patentamt
European Patent Office
Office européen des brevets

(11) Veröffentlichungsnummer: 0 002 535
B1

# (12) EUROPÄISCHE PATENTSCHRIFT

(45) Veröffentlichungstag der Patentschrift: 30.09.81

(51) Int. Cl.³: C 07 J 9/00, C 12 P 33/06

(21) Anmeldenummer: 78101711.6

(22) Anmeldetag: 15.12.78

## (54) Verfahren zur Herstellung von 21-Hydroxy-20-methyl-pregnan-Derivaten.

(30) Priorität: 19.12.77 DE 2757156

(43) Veröffentlichungstag der Anmeldung:
27.06.79 Patentblatt 79/13

(45) Bekanntmachung des Hinweises auf die Patenterteilung:
30.09.81 Patentblatt 81/39

(84) Benannte Vertragsstaaten:
BE CH DE FR GB IT LU NL SE

(56) Entgegenhaltungen:
US - A - 3 684 657
US - A - 3 759 791

CHEMICAL ABSTRACTS, Vol. 76, Nr. 17, 24, April 1972, Zusammenfassung Nr. 97886y, Columbus, Ohio, USA, MARSHECK W.J. et. al., "Microbial degradation of sterols", Seite 322, linke Spalte
CHEMICAL ABSTRACTS, Vol. 85, Nr. 23, 6. Dezember 1976, Zusammenfassung Nr. 175500y, Columbus, Ohio, USA, CONNER A.H. et. al., "Microbial conversion of tall oil sterols to C19 steroids", Seite 376, rechte Spalte

(73) Patentinhaber: SCHERING AKTIENGESELLSCHAFT
Berlin und Bergkamen
Müllerstrasse 170/178 Postfach 65 0311
D-1000 Berlin 65 (DE)

(72) Erfinder: Weber, Alfred, Dr.
Brümmerstrasse 74
D-1000 Berlin 33 (DE)
Erfinder: Kennecke, Mario, Dr.
Taubertstrasse 31f
D-1000 Berlin 33 (DE)
Erfinder: Müller, Rudolf, Dr.
Corneliusstrasse 17
D-1000 Berlin 46 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 535 B1

Courier Press, Leamington Spa, England.

# Verfahren zur Herstellung von 21-Hydroxy-20-methyl-pregnan-Derivaten

Die Erfindung betrifft das in den Patentansprüchen gekennzeichnete Verfahren.

Es ist bekannt, daß Kulturen von Mycobacterium spec. NRRL B—3683 und NRRL B—3805 die Fähigkeit besitzen, aus Zoo- oder Phytosteroinen 21-Hydroxy-20-methyl-pregnan-Derivate der allgemeinen Formel I zu bilden (Applied. Microbiology *23*, 1972, 72 ff. und Applied and Envi omental Microbiology *32*, 1976, 310 ff.). Diese Verbindungen werden aber nur spurenweise oder in sehr geringen Ausbeuten gebildet, so daß das bekannte Verfahren technisch kaum anwendbar ist.

Mit Hilfe des erfindungsgemäßen Verfahrens lassen sich signifikant höhere Ausbeuten an 21-Hydroxy-20-methyl-pregnan-Derivaten der allgemeinen Formel I erzielen, als mittels des bekannten Verfahrens.

Geeignete Zoo- oder Phytosterine für die Fermentation sind beispielsweise Cholesterin, Stigmasterin, Campesterin, Brassicasterin oder die Sitosterine, die 3-Acylverbindungen — wie zum Beispiel die Acetate dieser Sterine oder die aus diesen Sterinen durch Oppenauer-Oxydation erhaltenen 3-Keto-$\Delta^4$-steroide, wie zum Beispiel 4-Cholesten-3-on, 4-Stigmasten-3-on, 4-Sitosten-3-on oder 4-Campesten-3-on.

Erfindungsgemäß wird die Fermentation in Gegenwart von Borationen oder organischen Borverbindungen durchgeführt. Geeignete Borverbindungen sind beispielsweise Borsäure, Natriummetaborat, Dinatriumtetraborat, Calziummetaborat oder Triphenylborat. Die Fermentation wird vorzugsweise in Gegenwart von 0,1% bis 0,5% Borationen liefernde Agenzien durchgeführt. Die Zugabe der Borationen liefernden Agenzien erfolgt vorzugsweise 10 bis 30 Stunden nach Fermentationsbeginn.

Bei der Zugabe der Borationen liefernden Agenzien ist darauf zu achten, daß der pH-Wert der Fermentationskultur in üblicher Weise auf einen Wert zwischen 6,0 und 8,0 eingestellt wird.

Ansonsten wird die Fermentation unter den üblichen Bedingungen durchgeführt. Die Mikroorganismen werden in einem geeigneten Nährmedium unter Belüften Submerskulturen angezüchtet. Dann setzt man den Kulturen das Substrat (in einem geeigneten Lösungsmittel gelöst oder vorzugsweise in emulgierter Form) zu und fermentiert, bis eine maximale Substratumwandlung erreicht ist (ca. 96—160 Stunden).

Geeignete Substratlösungsmittel sind beispielsweise Methanol, Äthanol, Glykolmonomethyläther, Dimethylformamid oder Dimethylsulfoxyd). Die Emulgierung des Substrates kann beispielsweise bewirkt werden, indem man diese in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxydaddukte oder Fettsäureester von Polyglykolen. Als geeignete Emulgatoren seien die handelsüblichen Netzmittel Tegin(R), Tagat(R) und Span(R) beispielsmäßig genannt.

Bei der Fermentation ermöglicht die Emulgierung der Substrate oft einen erhöhten Substratdurchsatz und somit eine Steigerung der Substratkonzentration. Es ist aber selbstverständlich auch möglich, bei dem erfindungsgemäßen Verfahren andere Methoden zur Steigerung des Substratdurchsatzes, wie sie dem Fermentationsfachmann wohl bekannt sind, anzuwenden.

Die optimale Substratkonzentration, Substratzugabezeit und Fermentationsdauer ist von der Struktur des verwendeten Substrates abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Eine weitere Steigerung an Verfahrensprodukten läßt sich erzielen, wenn man die Mycobacterien Species in üblicher Weise selektiert oder mutiert.

So kann man beispielsweise Mycobacterium spec. NRRL B—3805 — zweckmäßigerweise nach Behandeln mit Mutagenen — auf Blut-Agar-Platten ausplatten und erhält Einzelkolonien von unterschiedlichen morphologischen Aussehen. Isoliert man diese Einzelkolonien und prüft sie auf ihre Fähigkeit zur Bildung von 21-Hydroxy-20-methyl-pregnan-Derivaten der allgemeinen Formel I, so findet man, daß insbesondere unter den Selektionsstämmen, die runde Kolonien bilden, oft Stämme sind, die eine 1,5 bis 3-fach höhere Ausbeute an 21-Hydroxy-20-methyl-pregnan-Derivate bilden als der nichtselektierte Stamm.

Die 21-Hydroxy-20-methyl-pregnan-Derivate der allgemeinen Formel I sind wertvolle Zwischenprodukte zur Synthese pharmakologisch wirksamer Steroide. So ist es beispielsweise möglich, diese Verbindungen zu den entsprechenden Pregnan-20-carbonsäuren der allgemeinen Formel III zu oxydieren, welche nach dem von H. Ruschig et al. (Chem. Ber. *88*, 1955, 883 ff) beschriebenen Verfahren in die entsprechenden Pregnan-3.20-dion-Derivate der Formel IV überführt werden können.

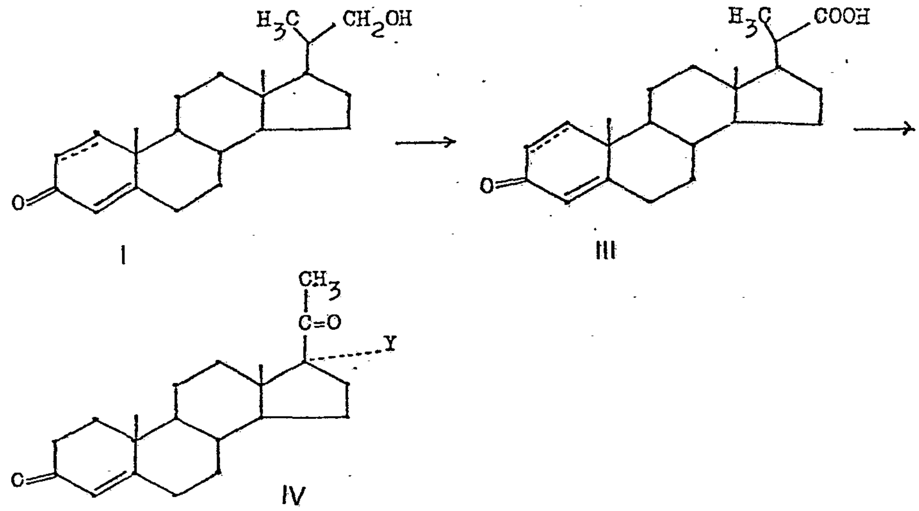

I III

IV

(Y = Wasserstoff oder 17$\alpha$-Hydroxy)

Die so erhaltenen Verbindungen zeichnen sich bekanntlich durch ihre gestagene Wirksamkeit aus und sind darüberhinaus wertvolle Zwischenprodukte zur Synthese zahlreicher pharmakologisch wirksamer Steroide.

Die nachfolgenden Beispiele dienen zur näheren Erläuterung der Erfindung.

Beispiel 1

a) Ein 2 l Erlenmeyerkolben mit 500 ml sterilem Nährmedium enthaltend

1 % Hefeextrakt
0,45% Dinatriumhydrogenphosphat
0,34% Kaliumdihydrogenphosphat
0,2 % Tagat(R)02
— eingestellt auf pH 6,7 —

wird mit einer Abschwemmung einer Trockenkultur von Mycobacterium spec. NRRL B—3805 beimpft und 3 Tage mit 190 Umdrehungen pro Minute bei 30°C geschüttelt.

b) 22 g Sitosterin werden mit 4,4 g Tegin(R) und 430 ml Wasser bei 95°C mit einem Ultra-Turrax(R) (Firma Jahnke u. Künkel) 25 Minuten emulgiert und anschließend auf 513 g mit Wasser aufgefüllt. Man sterilisiert die Emulsion 20 Minuten lang bei 120°C.

c) Ein 500 ml Erlenmeyerkolben mit 65 ml sterilem Nährmedium — enthaltend

2 g Cornsteep liquor
0,3 g Diammoniumhydrogenphosphat
0,25 g Tagat(R)02
— eingestellt auf pH 6,5 —

wird mit 5 ml der Microbacterium spec. Anzuchtskultur beimpft.

Dann werden 28 ml der unter Ib) hergestellten Suspension (dies entsprecht 1,2 g Sitosterin) und nach 24 Stunden 4 ml 4%ige wässrige Natriumtetraboratlösung zugegeben und weitere 120 Stunden lang bei 30°C unter Schütteln fermentiert.

Nach erfolgter Fermentation wird die Kulturbrühe 2 mal mit je 100 Äthylenchlorid extrahiert. Die vereinigten Äthylenchloridextrakte werden sodann mit 11 g Aktivkohle versetzt und über ein Faltenfilter filtriert. Das Filtrat wird anschließend bei 40°C im Rotationsverdampfer eingeengt und an Aluminiumoxid chromatographiert. Nach erfolgter Chromatographie erhält man 135 mg 21-Hydroxy-20-methyl-4-pregnen-3-on vom Schmelzpunkt 140—141°C (aus Äthylacetat).

Führt man die Reaktion unter den gleichen Bedingungen, aber ohne Zusatz von Natriumtetraboratlösung durch, so erhält man 45 mg 21-Hydroxy-20-methyl-4-pregnan-3-on.

Beispiel 2

a) Aus 22 g 4-Cholesten-3-on wird wie im Beispiel 1b beschrieben 513 g Emulsion hergestellt.

b) Unter den Bedingungen des Beispiels 1c werden 70 ml einer Mycobacterium spec. NRRL B—3805 Kultur angezüchtet, mit 28 ml der 4-Cholesten-3-on-Emulsion und nach 24 Stunden mit 4 ml 4%iger wässriger Natriumtetraboratlösung versetzt und weitere 120 Stunden lang bei 30°C unter Schütteln fermentiert.

Man arbeitet den Fermentationsansatz auf, wie im Beispiel 1c beschrieben und erhält 95 mg 21-Hydroxy-20-methyl-4-pregnen-3-on vom Schmelzpunkt 142—144°C.

Führt man die Reaktion unter den gleichen Bedingungen, aber ohne Zusatz von Natriumtetraboratlösung aus, so erhält man 40 mg 21-Hydroxy-20-methyl-4-pregnen-3-on.

Beispiel 3

28 ml der gemäß Beispiel 1b hergestellten Sitosterin-Emulsion werden wie im Beispiel 1c beschrieben mit 70 ml einer Mycobacterium spec. NRRL B—3805 Kultur fermentiert, wobei man an Stelle der wässrigen Natriumtetraboratlösung einen Zusatz von 6 ml 4%iger Calziummetaboratsuspension in Wasser verwendet.

Nach Aufarbeitung des Fermentationsansatzes wie im Beispiel 1c beschrieben, erhält man 120 mg 21-Hydroxy-20-methyl-4-pregnen-3-on vom Schmelzpunkt 141—143°C.

Beispiel 4

28 ml der gemäß Beispiel 1b hergestellten Sitosterin-Emulsion werden wie im Beispiel 1c beschrieben mit 70 ml einer Mycobacterium spec. NRRL B—3805 Kultur fermentiert, wobei man an Stelle der wässrigen Natriumtetraboratlösung einen Zusatz von 5 ml einer 4%igen Triphenylboratlösung verwendet.

Nach Aufarbeitung des Fermentationsansatzes wie im Beispiel 1c beschrieben, erhält man 125 mg 21-Hydroxy-20-methyl-4-pregnen-3-on vom Schmelzpunkt 142—143°C.

Beispiel 5

a) Unter den Bedingungen des Beispiels 1a wird eine Mycobacterium spec. NRRL B—3683 Kultur angezüchtet.

b) 65 ml des im Beispiel 1c beschriebenen Nährmediums werden mit 5 ml der Mycobacterium spec. NRRL B—3683 Anzuchtskultur versetzt. Dann setzt man von der Kultur 28 ml der im Beispiel 1b beschriebenen Sitosterin-Emulsion und nach 24 Stunden 4 ml 4%ige Natriumtetraboratlösung zu und fermentiert weitere 120 Stunden lang. Man arbeitet den Fermentationsansatz auf, wie im Beispiel 1c beschrieben und erhält 90 mg 21-Hydroxy-20-methyl-1.4-pregnadien-3-on vom Schmelzpunkt 180—182°C (aus Äthylacetat-Aceton).

Beispiel 6

40 ml einer Anzuchtskultur von Mycobacterium spec. NRRL B—3805 — hergestellt nach Beispiel 1a — werden mit 4000 Umdrehungen pro Minute zentrifugiert.

Die so erhaltene Bakterienmasse wird dann zweimal mit einer auf pH 6 abgepufferten Salzlösung enthaltend 0,5% Natriumchlorid, 0,012% Magnesiumsulfat (Heptahydrat) und 1,36% Kaliumdihydrogenphosphat gewaschen, in 40 ml dieser Salzlösung suspendiert und mit 10 ml einer 0,5%igen 1-Methyl-3-nitro-1-nitrosoguanidin versetzt.

Man inkubiert die Bakteriensuspension eine Stunde lang bei 30°C, zentrifugiert die Bakterien ab, wäscht sie zweimal mit der obengenannten Salzlösung und plattet sie auf Blut-Agar-Platten (Herstellungsfirma Oxoid Ltd., London) aus. Von den gebildeten Einzelkolonien werden jeweils diejenigen ausgewählt, die runde Kolonien bilden und aus diesen jeweils Anzuchtskulturen hergestellt, wie im Beispiel 1a beschrieben. Die Anzuchtskolonien dienen jeweils dazu, die im Beispiel 1c beschriebene Fermentation durchzuführen, mit folgendem Ergebnis:

| Anzahl der getesteten Selektionsstämme | Ausbeute an 21-Hydroxy-20-methyl-4-pregnen-3-on ohne Zusatz von Natriumtetraborat | mit Zusatz von Natriumtetraborat |
|---|---|---|
| 19 | 0—200 mg | — |
| 8 | 201—400 mg | — |
| 94 | 401—600 mg | — |
| 35 | 601—800 mg | — |
| 7 | 801—1000 mg | 1550—2100 mg |
| 0 | 1001—1020 mg | — |
| 6 | 1021—1040 mg | 1900—3000 mg |
| 1 | 1041—1060 mg (150 mg) | 2900 mg |
| 0 | 1061—1080 mg | — |
| 1 | 1081—2000 mg (195 mg) | 3450 mg |

**Patentansprüche**

1. Verfahren zur Herstellung von 21-Hydroxy-20-methylpregnan-Derivaten der allgemeinen Formel I

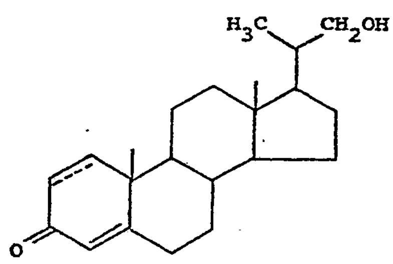

(I),

worin die Bindung ..... eine Einfachbindung oder eine Doppelbindung bedeutet, durch Fermentation von Zoo- oder Phytosterinen mit einer Kultur von Mycobacterium spec. NRRL B—3683 oder NRRL B—3805 oder seiner Varianten und Mutanten, dadurch gekennzeichnet, daß man die Fermentation bei einem pH-Wert von 6,0 bis 8,0 in Gegenwart von Borationen oder organischen Borverbindungen durchführt.

2. Verfahren nach Anspruch 1, dadurch gekennzeichnet, daß man ein Zoo- oder Phytosterin der allgemeinen Formel II

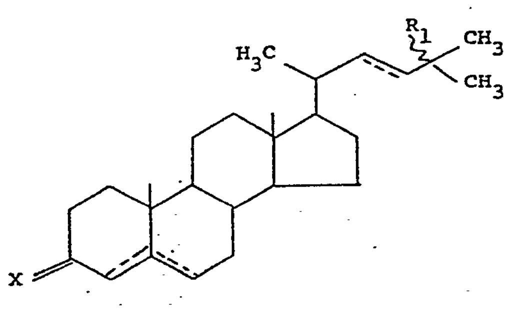

(II),

worin die Bindungen ..... Einfachbindungen oder Doppelbindungen, X eine Oxogruppe oder ein Wasserstoffatom und $R_1$ ein Wasserstoffatom, eine Methylgruppe oder eine Äthylgruppe darstelle, fermentiert.

3. Verfahren nach Anspruch 1 und 2, dadurch gekennzeichnet, daß man als Borationen lieferndes Agens Orthoborsäure, Metaborsäure, Polyborsäure oder deren Alkali- oder Erdalkalimetallsalze verwendet.

4. Verfahren nach Anspruch 1 bis 3, dadurch gekennzeichnet, daß man als organische Borverbindung Triphenylborat verwendet.

5. Verfahren nach Anspruch 3, dadurch gekennzeichnet, daß man die Fermentation in Gegenwart von 0,1% bis 0,5% eines Borationen liefernden Agens durchführt.

6. Verfahren nach Anspruch 5, dadurch gekennzeichnet, daß man das Borationen lieferende Agens 10 bis 30 Stunden nach Fermentationsbeginn zusetzt.

**Revendications**

1. Procédé de préparation des dérivés de l' hydroxy-21-méthyle-20-piegnane qui répondent à la formule générale I

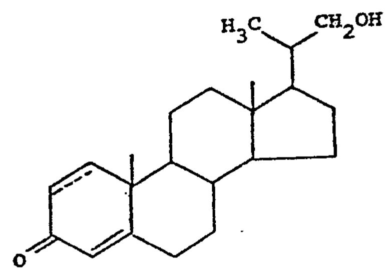

(I),

dans laquelle la liaison ..... représente une liaison simple ou une liaison double caractérisé en ce que l'on fait — au moyen de la fermentation de zoostérols ou de phytostérols avec une culture de Mycobacterium spec. NRRL B—3683 ou NRRL B—3805 ou ses variantes et mutations — fermenter à un pH de 6,0 à 8,0 en présence d'ions de borate ou de composés boriques organiques.

2. Procédé selon la revendication 1, caractérisé en ce que l'on fait fermenter un zoostérol ou phytostérol répondant à la formule II

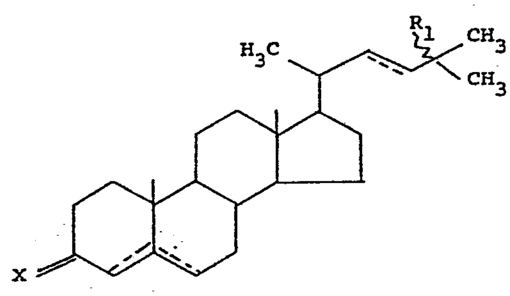

(II),

dans laquelle les liaisons ..... représentent des liaisons simples ou des liaisons doubles

X représente un atome d'oxygène ou un atome d'hydrogène. et un groupe hydroxy

$R_1$ représente un atome d'hydrogène, un groupe de méthyle ou un groupe d'éthyle.

3. Procédé selon les revendications 1 et 2, caractérisé en ce que l'on utilise en tant qu'agent chimique fournissant les ions de borate de l'acide orthoborique, de l'acide métaborique ou de l'acide polyborique ou leurs sels métalliques alcalins ou alcalino-terreux.

4. Procédé selon les revendications 1 à 3, caractérisé en ce que l'on utilise du triphénylborate en tant que composé borique organique.

5. Procédé selon la revendication 3, caractérisé en ce que l'on fait fermenter en présence de 0,1% à 0,5% d'un agent chimique fournissant les ions de borate.

6. Procédé selon la revendication 5, caractérisé en ce que l'on ajoute l'agent chimique fournissant les ions de borate 10 à 30 heures après le début de la fermentation.

**Claims**

1. Process for the manufacture of 21-hydroxy-20-methyl-pregnane derivatives of the general formula I

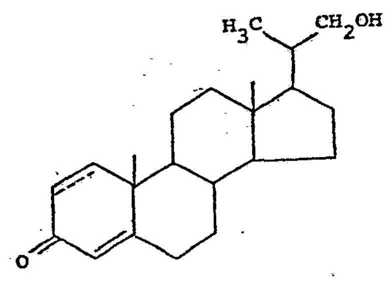

(I),

in which the bond ..... denotes a single bond or a double bond, by fermenting zoo- or phyto-sterols with a culture of *Mycobacterium spec.* NRRL B—3683 or NRRL B—3805 or variants and mutants thereof, characterised in that the fermentation is carried out at a pH of from 6.0 to 8.0 in the presence of borate ions or organic boron compounds.

2. Process according to claim 1, characterised in that a zoo- or phyto-sterol of the general formula II

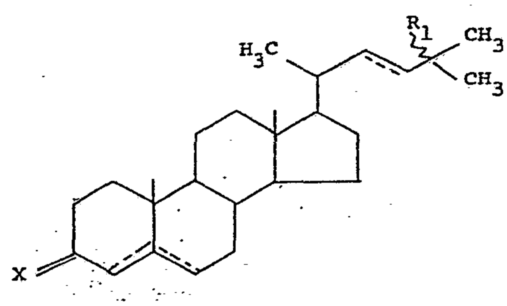

(II),

is fermented, in which the bonds ..... represent single bonds or double bonds, X represents an oxo group or a hydrogen atom and $R_1$ represents a hydrogen atom, a methyl group or an ethyl group.

3. Process according to claims 1 and 2, characterised in that orthoboric acid, metaboric acid, polyboric acid or the alkali metal or alkaline earth metal salts thereof are used as the agent that yields borate ions.

4. Process according to claims 1 to 3, characterised in that triphenyl borate is used as the organic boron compound.

5. Process according to claim 3, characterised in that the fermentation is carried out in the presence of from 0.1% to 0.5% of an agent that yields borate ions.

6. Process according to claim 5, characterised in that the agent that yields borate ions is added from 10 to 30 hours after the onset of fermentation.